# EUROPÄISCHE PATENTANMELDUNG

(11) **EP 0 535 420 A1**
(43) Veröffentlichungstag der Anmeldung: **07.04.1993**
(21) Anmeldenummer: 92115542.0
(22) Anmeldetag: 11.09.1992
(51) Int. Cl.: C07D 403/10, A61K 31/415, A61K 31/41

(54) **Acylale von Imidazol-5-carbonsäurederivaten, als Angiotensin II Inhibitoren**

(30) Priorität: 04.10.1991 AT 1987/91
(71) Anmelder: Chemisch Pharmazeutische Forschungsgesellschaft m.b.H., A-4021 Linz (AT)
(72) Erfinder: Binder, Dieter, Dr., A-1190 Wien (AT); Weinberger, Josef, Dr., A-4540 Bad Hall (AT)

(57) **Zusammenfassung**

Neue Imidazol-5-carbonsäureacylale der allgemeinen Formel 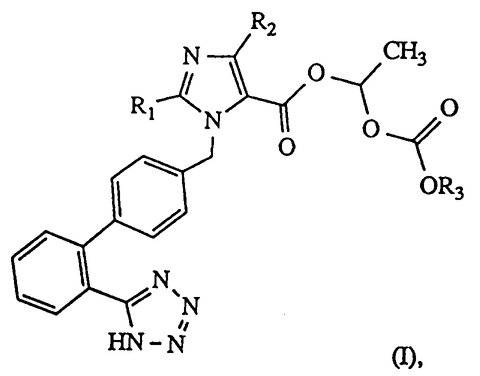
worin R₁ eine gegebenenfalls ungesättigte, geradkettige Alkylgruppe mit 1 - 6 Kohlenstoffatomen und R₂ Wasserstoff, Chlor, Brom, Jod oder CF₃ und R₃ C₁-C₁₀-Alkyl, C₃-C₇-Cycloalkyl oder Benzyl bedeuten und ihre pharmazeutisch verträglichen Salze.

## Beschreibung

Die Erfindung betrifft neue therapeutisch wertvolle Acylale von Imidazol-5-carbonsäurederivaten und ihre Salze, ein Verfahren zu ihrer Herstellung und ihre Verwendung in Medikamenten mit blutdrucksenkender Wirkung.

Es ist bereits eine Vielzahl von Verbindungen bekannt, die zur Behandlung von Bluthochdruck, verursacht durch Angiotensin II, verwendet werden können.

Ein bekannter Angiotensin II Rezeptor Antagonist, DuP 753 (2n-Butyl-4-chloro-5-hydroxymethyl-1-((2'-1-H-tetrazol-5-yl)biphenyl-4-yl)methyl)imidazol) wird beispielsweise in Journal of Pharmacology and Experimental Therapeutics, P.C. Wong et al. 1990, Vol 225, S 211 - 217 beschrieben. DuP 753 wird jedoch bei der in vivo Anwendung zu einem nicht kompetitiven Metaboliten EXP 3174, (2-n-Butyl-4-chloro-1-((2'-(1H-tetrazol-5-yl)biphenyl-4-yl)methyl)imidazol-5-carbon-säure), umgewandelt, der zu einem Großteil für die Wirkungsdauer von DuP 753 verantwortlich ist. Der Nachteil von nicht kompetitiven Antagonisten ist jedoch, daß sie irreversibel an den Rezeptor gebunden werden und dort Änderungen der zellulären Struktur auslösen.

In der EP-A1-0253310 werden unter anderem Angiotensin II rezeptorblockende Imidazolcarbonsäuren der Formel 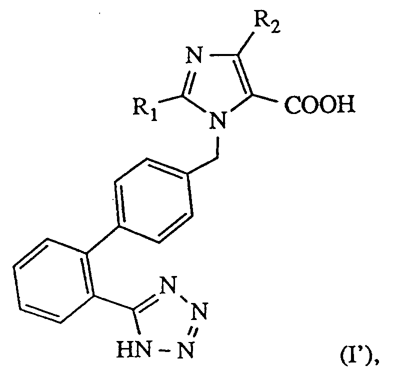
worin R₁ eine gegebenenfalls ungesättigte, geradkettige Alkylgruppe mit 1 - 6 Kohlenstoffatomen und R₂ Wasserstoff, Chlor, Brom, Jod und CF₃ bedeuten kann, geoffenbart, die sich durch besonders starke Wirkung auszeichnen. Diese Verbindungen zeigen bei intravenöser Verabreichung hervorragende blutdrucksenkende Wirkung. Ihr Nachteil ist, daß sie bei oraler Verabreichung nur zu einem geringen Teil resorbiert werden und eine geringere Wirkungsstärke erreichen oder höher dosiert werden müssen.

Aufgabe der vorliegenden Erfindung war es somit rein kompetitive Antagonisten zu finden, die bei oraler Verabreichung eine mehrfach bessere Resorption und damit eine höhere Wirksamkeit als die Carbonsäuren der allgemeinen Formel (I') aufweisen und schon bei der Passage durch den Darm im Blut wieder als freie Carbonsäuren vorliegen. Diese Aufgabe konnte nun unerwarteterweise mit den erfindungsgemäßen Acylalen und Estern gelöst werden.

Gegenstand der vorliegenden Erfindung sind somit neue Verbindungen der allgemeinem Formel 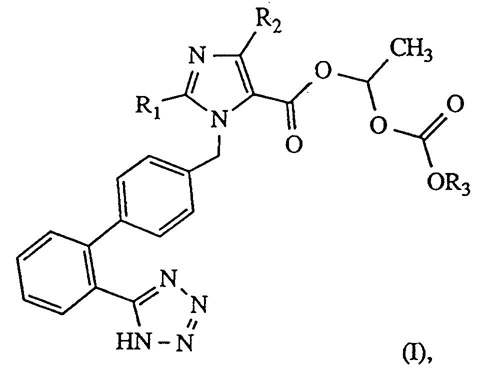
worin R₁ eine gegebenenfalls ungesättigte, geradkettige Alkylgruppe mit 1 - 6 Kohlenstoffatomen, R₂ Wasserstoff, Chlor, Brom, Jod oder CF₃ und R₃ C₁-C₁₀-Alkyl, C₃-C₇-Cycloalkyl oder Benzyl bedeuten sowie ihre pharmazeutisch verträgliche Salze.

Eine bevorzugte Klasse von Verbindungen sind jene in denen R₁ Butyl, R₂ Chlor und R₃ Ethyl bedeuten.

Ein weiterer Gegenstand der vorliegenden Erfindung ist ein Verfahren zur Herstellung der neuen Verbindungen der Formel (I), worin R₁, R₂ und R₃ die obige Bedeutung haben, das darin besteht, daß man im Schritt a) eine Verbindung der Formel 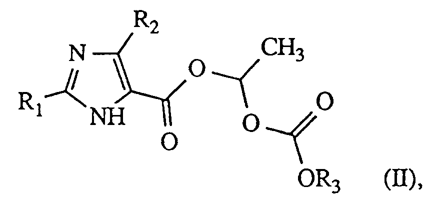
worin R₁, R₂ und R₃ die obige Bedeutung haben mit einer Verbindung der Formel 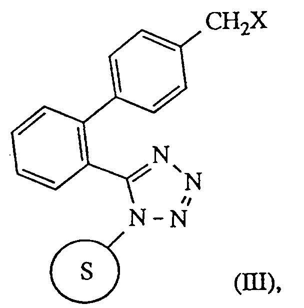
worin X Chlor, Brom oder Jod und 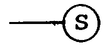
die Triphenylmethylschutzgruppe bedeutet, umsetzt, die so erhaltene Verbindung der Formel 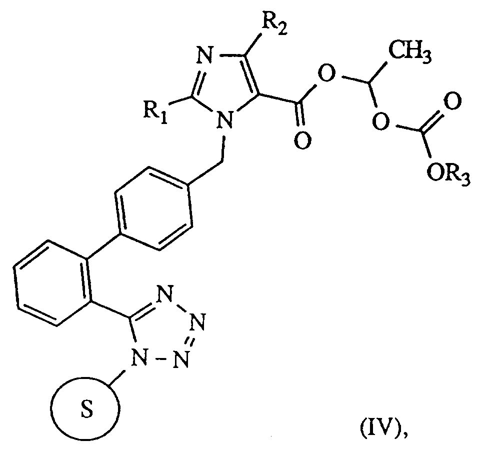
worin R₁, R₂, R₃ und 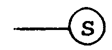
die obige Bedeutung besitzen, im Schritt b) mit einem niederen aliphatischen Alkohol erhitzt und die so erhaltene Verbindung der Formel (I), die wegen ihres amorphen Charakters üblicherweise nicht kristallisiert, gegebenenfalls mit anorganischen oder organischen Basen in ein pharmazeutisch verträgliches, kristallines Salz überführt.

Die erfindungsgemäße Umsetzung wird im Schritt a) am besten so durchgeführt, daß man eine Lösung der Verbindungen der allgemeinen Formel II und III in einem reaktionsinerten, wasserfreien, organischen Lösungsmittel, wie z. B. Ether, Dioxan, THF, Aceton, Dimethylformamid oder Dimethylsulfoxid in Gegenwart von einem Äquivalent festem Kaliumcarbonat erwärmt. Die günstigste Reaktionstemperatur liegt dabei zwischen 20 und 100°C, die Reaktionszeit beträgt abhängig davon 0,5 bis 20 Sekunden.

Die, im Schritt b), folgende Abspaltung der TriphenylmethylSchutzgruppe 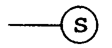
aus den erhaltenen Verbindungen der allgemeinen Formel IV erfolgt durch Sieden in einem niederen aliphatischen Alkohol, wie z. B. Methanol oder Ethanol, die Reaktionszeit beträgt dabei zwischen 5 Minuten und 10 Stunden. Die bei der Umsetzung im Verfahrensschritt b) erhaltenen Verbindungen der allgemeinen Formel (I) können auf übliche Weise mit anorganischen und organischen Basen in ihre pharmazeutisch verwendbaren Salze übergeführt werden. Die Salzbildung kann beispielsweise durchgeführt werden, indem man die genannten Verbindungen der Formel (I) in einem geeigneten Lösungsmittel z. B. Wasser, einem niederen aliphatischen Alkohol, THF, Dioxan, Benzol, CH₂Cl₂, CHCl₃, Diethylether, DMF oder DMSO, löst, eine äquivalente Menge der gewünschten Base zusetzt, für eine gute Durchmischung sorgt und nach beendeter Salzbildung das Lösungsmittel im Vakuum abzieht. Gegebenenfalls können die Salze nach der Isolierung umkristallisiert werden.

Pharmazeutisch verwendbare Salze sind z. B. Metallsalze, insbesonders Alkalimetall- oder Erdalkalimetallsalze, wie Natrium-, Kalium-, Magnesium- oder Calciumsalze. Andere pharmazeutische Salze sind beispielsweise leicht kristallisierende Ammoniumsalze. Letztere leiten sich von Ammoniak oder organischen Aminen, z. B. Mono-, Di-, oder Tri-nieder-(alkyl, cycloalkyl oder hydroxyalkyl)-aminen, Niederalkylendiaminen oder (Hydroxy-niederalkyl oder Aryl-niederalkylammoniumbasen, z. B. Methylamin, Diethylamin, Triethylamin, Dicyclohexylamin, Triethanolamin, Ethylendiamin, Tris(hydroxymethyl)-aminomethan, Benzyltrimethylammoniumhydroxyd und dergleichen ab.

Die Verbindungen der allgemeinen Formel (II) können ausgehend von Verbindungen der Formel (V), worin R₁ und R₂ die obige Bedeutung haben, gemäß dem folgenden Reaktionsschema nach üblichen und dem Fachmann geläufigen chemischen Arbeitsmethoden hergestellt werden. 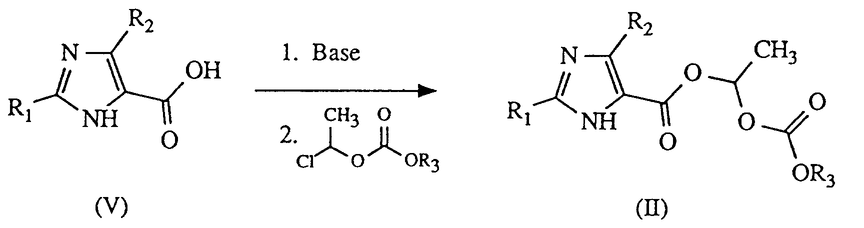
Die Verbindungen der allgemeinen Formeln (III) und (V) sind literaturbekannt (D.J.Carini et al. EP 0 324 377, 1989).

Die neuen Verbindungen der allgemeinen Formel I und ihre Salze sind oral wirksam und unterdrücken die vasokonstriktive und blutdrucksteigende Wirkung von Angiotensin II und zeigen in Tiermodellen hervorragende blutdrucksenkende Wirkung.

Aufgrund dieser pharmakologischen Eigenschaften können die neuen Verbindungen allein oder in Mischung mit anderen Wirksubstanzen in Form üblicher galenischer Zubereitung als Heilmittel zur Behandlung von Bluthochdruck und anderen cardiovaskulären Erkrankungen Verwendung finden.

Die Erfindung bezieht sich daher weiterhin auf Arzneimittel, die die erfindungsgemäßen Verbindungen der allgemeinen Formel (I) oder ihre Salze, als blutdrucksendende Wirksubstanz, allein enthalten oder in Mischung mit anderen Wirksubstanzen in Form üblicher oraler, galenischer Zusammensetzung. Die erfindungsgemäßen Verbindungen können in Form von Tabletten oder Kapslen, welche eine Einheitsdosierung der Verbindung zusammen mit Verdünnungsmitteln, wie Maisstärke, Calciumcarbonat, Dicalciumphosphat, Alginsäure, Lactose, Magnesiumstearat, Primogel oder Talkum erhalten, oral appliziert werden. Die Tabletten werden in herkömmlicher Weise durch Granulieren der Inhaltsstoffe und Pressen, die Kapseln durch Einfüllen in Hartgelatinekapseln geeigneter Größe, hergestellt.
Für die orale Applikation beim Menschen wird der tägliche Dosierungswert einer erfindungsgemäßen Verbindung im Bereich von 0,1 bis 30 mg/kg pro Tag für einen typischen erwachsenen Patienten von 70 kg liegen. Daher können Tabletten oder Kapseln üblicherweise 0,1 bis 50 mg an aktiver Verbindung für die orale Applikation bis zu dreimal am Tag erhalten.
Selbstverständlich wird aber der Artz in jedem Fall die tatsächliche Dosierung bestimmen, welche für den individuellen Patienten am geeignetsten ist, wobei diese mit dem Alter, der Masse und dem Ansprechen des Patienten variieren kann.

### Beispiel 1:

### 2-Butyl-4-chlor-1-((2'-(1H-tetrazol-5-yl)-biphenyl-4-yl)-methyl)-1H-imidazol-5-carbonsäure-1-ethoxycarbonyloxyethylester 8,0 g (10,06 mMol)

2-Butyl-4-chlor-1-((2'-(N-triphenyl-1H-tetrazol-5-yl)-biphenyl-4-yl)-methyl)-1H-imidazolcarbonsäure-1-ethoxycarbonyloxyethylester werden in 175 ml Methanol 3 Stunden zum Sieden erhitzt, das Lösungsmittel wird abgezogen und das erhaltene Rohprodukt einer säulenchromatographischen Trennung unterworfen (Et₂O; 400 g KG 60).
Ausbeute: 5,0 g farblose amorphe Substanz

| Mikroelementaranalyse: C₂₇H₂₉C1N₆O₅ MG: 553,02 | | | |
|---|---|---|---|
| | C | H | N |
| berechnet | 58,64 | 5,29 | 15,29 |
| gefunden | 58,3 | 5,4 | 15,3 |

**¹H-NMR:** (CDCl₃)
δ (ppm): 7,91 (dd, 1H, Biph-H3'); 7,64-7,43 (m, 2H,Biph-H4', H5'); 7,41 (dd, 1H,Biph-H6'); 7,10 (AA'; 2H, Biph-H3, H5); 6,89 (BB',2H, Biph-H2, H6); 6,82 (q,1H, CH-CH₃); 5,48 (s, 2H, Biph-CH₂); 4,15 (q, 2H, -CH₂-CH₃); 2,66 (t, 2H, Bu1-CH₂-); 1,63 (m, 2H, Bu2-CH₂-); 1,54 (d, 3H, CH-CH₃); 1,32 (m, 2H, Bu3-CH2-); 1,21 (t, 3H, -CH₂-CH₃); 0,86 (t, 3Hm Bu4-CH₃)
**¹³C-NMR:** (CDCl₃)
δ (ppm): 156,71; 156,59; 153,20; 152,37; 140,63; 139,21; 136,54; 135,23; 130,51; 130,37; 129,94; 129,18; 127,45; 125,94; 125,72; 115,74; 91,37; 64,16; 47,80; 28,58; 25,83; 21,53; 19,22, 13,85; 13,50

### Beispiel 2:

### 2-Butyl-4-chlor-1-((2'-(1H-tetrazol-5-yl)-biphenyl-4-yl)-methyl)-1H-imidazol-5-carbonsäure-1-ethoxycarbonyloxyethylester, Natriumsalz

Zu 2,0 g (3,62 mMol) 2-Butyl-4-chlor-1-((2'-(1H-tetrazol-5-yl)-biphenyl-4-yl)-methyl)-1H-imidazol-5-carbonsäurelethoxycarbonyloxyethylester in 40 ml Dichlormethan werden 0,41 g (3,62 mMol) Natriumtrimethylsilanolat in 3 ml Dichlormethan getropft, 1 Stunde gerührt, das Lösungsmittel abgezogen und der Rückstand in Diisopropylether kristallisiert, filtriert und 3x mit kaltem Diisopropylether digeriert.
Ausbeute: 1,3 g farblose Kristalle
FP: Zers. ab 138°C

| Mikroelementaranalyse: C₂₇H₂₈ClN₆O₅Na.H₂O MG: 593,02 | | | |
|---|---|---|---|
| | C | H | N |
| berechnet | 54,69 | 5,10 | 14,17 |
| gefunden | 54,47 | 5,08 | 14,28 |

**¹H-NMR:** DMSO)
δ (ppm): 7,56 (dd, 1H, Biph-H3'); 7,41 - 7,23 (m, 3H, Biph-H4',H5',H6'); 7,10 (AA', 2H, Biph-H3,H5); 6,86 (BB', 2H, Biph-H2, H6); 6,78 (q, 1H, CH-CH₃); 5,52 (AB,2H, Biph-CH₂); 4,13 (q, 2H, -CH₂-CH₃); 2,65 (t, 2H, BulCH₂-); 1,56 (m, 2H, Bu2-CH₂-); 1,48 (d, 3H, CH-CH₃); 1,29 (m, 2H, Bu3-CH₂-); 1,19 (t, 3H,-CH₂-CH₃); 0,82 (t, 3H, Bu4-CH₃)
**¹³C-NMR:** (DMSO)
δ (ppm): 160,66; 156,72; 153,19; 152,36; 141,20; 139,74; 136,52; 134,10; 132,54; 130,46; 130,00; 129,40; 127,21; 126,67; 125,14; 115,68; 91,38; 64,16; 47,84; 28,62; 25,87; 21,56; 19,27; 13,88; 13,58
Das Ausgangsmaterial kann wie folgt hergestellt werden:

### 2-Butyl-4-chlor-1H-imidazol-5-carbonsäure-1-ethoxycarbonyloxyethylester

Zu 7,66 g (37,80 mMol) 2-Butyl-4-chlor-1H-imidazol-5-carbonsäure in 155 ml Hexamethylphosphorsäuretriamid werden 4,88 g (43,47 mMol) Natriumsilanolat in 20 ml THF getropft und 30 Minuten gerührt.
Zu dieser Lösung werden 6,34 g (41,58 mMol) Chlorethylethylcarbonat in 25 ml Hexamethylphosphorsäuretriamid getropft und eine Stunde auf 80°C gehalten. Das Reaktionsgemisch wird auf 760 ml Wasser gegossen, mit 8 x 75 ml Diethylether extrahiert, die vereinten organischen Phasen werden mit 3 x 65 ml wäßriger Natriumhydrogencarbonatlösung und mit 3 x 100 m1 Wasser gewaschen. Die organische Phase wird über Natriumsulfat/Aktivkohle getrocknet, filtriert und das Lösungsmittel abgezogen. Das erhaltene Rohprodukt wird einer säulenchromatographischen Trennung unterworfen
(EE:CH₂Cl₂ = 1:25; 300 g KG 60) Ausbeute: 7,4 g farblose Kristalle
FP: 108 - 110°C

| Mikroelementaranalyse: C₁₃H₁₉C N₂O₅ MG:318,76 | | | |
|---|---|---|---|
| | C | H | N |
| berechnet | 48,99 | 6,01 | 8,79 |
| gefunden | 48,84 | 5,86 | 8,65 |

**¹H-NMR:** (CDCl₃)
δ (ppm): 6,92 (q, 1H, CH-CH₃); 4,18 (q, 2H, -CH₂-CH₃); 2,71 (t, 2H, Bu1-CH₂-); 1,69 (m, 2H, Bu2-CH₂-); 1,57 (d, 3H, CH-CH₃); 1,43 (m, 2H, Bu3-CH₂-); 1,28 (t, 3H, -CH2-CH₃); 0,86 (t, 3H, Bu4-CH₃)
**¹³C-NMR:** (CDCl₃)
δ (ppm): 157,48; 152,95; 141,91; 136,71; 115,73; 91,47; 64,57 29,90; 28,28; 22,13; 19,56; 14,00; 13,53

### 2-Butyl-4-chlor-1-((2'-(N-triphenyl-1H-tetrazol-5-yl)-biphen4-yl)methyl)-1H-imidazolcarbonsäure-1-ethoxycarbonyloxyethylester

7,1 g (22,27 mMol) 2-Butyl-4-chlor-1H-imidazol-5-carbonsäure-1-ethoxycarbonyloxyethylester, 15,52 g (27,84 mMol) N-Triphenylmethyl-5-(4'-brommethyl-biphenyl-2-yl)-1H-tetrazol und 3,85 g (27,84 mMol) Kaliumcarbonat werden in 230 ml DMF 1,5 Stunden bei 70°C gerührt. Nach Abziehen des Lösungsmittels wird der Rückstand zwischen 250 ml halbkonz. Ammoniumchloridlösung und 100 ml Diethylether verteilt, die Phasen getrennt und die Wäßrige mit 4 x 50 ml Diethylether extrahiert. Die vereinten organischen Phasen werden mit 5 x 50 ml Wasser gewaschen, über Natriumsulfat/Aktivkohle getrocknet, filtriert und das Lösungsmittel abgezogen. Das erhaltene Rohprodukt wird einer säulenchromatographischen Trennung unterworfen (Bz:Et₂O=6:1; 400 g KG 60)
Ausbeute: 9,65 g farblose Kristalle
FP: 150 - 153°C

| Mikroelementaranalyse: C₄₆H₄₃C1N₆O₅ MG: 795,34 | | | |
|---|---|---|---|
| | C | H | N |
| berechnet | 69,47 | 5,45 | 10,57 |
| gefunden | 69,39 | 5,65 | 10,74 |

**¹H-NMR:** (DMSO)
δ (ppm): 7,92 (dd, 1H, Biph-H3'); 7,54-7,39 (m, 2H, Biph-H5', H6'); 7,39-7,23 (m, 9H, Trit-H2, H4, H6); 7,21 (t, 1H, Biph-H4'); 7,10 (AA', 2H, Biph-H3, H5); 6,98-6,90 (m, 6H, Trit-H3, H5); 6,88 (q, 1H, CH-CH₃); 6,81 (BB', 2H, Biph-H2, H6); 5,45 (AB, 2H, Biph-CH₂); 4,19 (q, 2H, -CH₂-CH₃); 2,50 (t, 2H, Bu1-CH₂-); 1,64 (m 2H, Bu2-CH₂-); 1,53 (d, 3H, CH-CH₃); 1,28 (m, 2H, Bu3-CH₂-); 1,27 (t, 3H, -CH₂-CH₃); 0,86 (t, 3H, Bu4-CH₃)
**¹³C-NMR:** (DMSO)
δ (ppm): 163,84; 147,40; 153,03; 152,92; 141,29; 141,13; 140,13; 140,64; 138,05; 134,39; 130,67; 130,19; 130,09; 129,87; 129,66; 128,17; 127,73; 127,54; 126,13; 125,53; 116,12; 91,28; 82,78; 64,34; 48,20; 29,15; 26,72; 22,17; 19,47; 14,02; 13,60

### Beispiel 3:

Die Affinität der Substanzen zum Angiotensin II-1 Subtyp Rezeptor wurde an Nebennierenrindenmikrosomen von Ratten (System: ³H-DuP 753) ermittelt.

Mit einer IC₅₀ von 79.4 nmol/l zeigte die Verbindung gemäß Beispiel 1 geringere Affinität als DuP 753 (7,24 nmol/l) und EXP 3174 (7,87 nmol/l).
Untersuchungen an der isolierten Rattenaorta haben gezeigt, daß die Verbindung gemäß Beispiel 1 und EXP 3174 selektive, nicht kompetitive Angiotensin II Rezeptor Antagonisten darstellen. (Abb. 1) 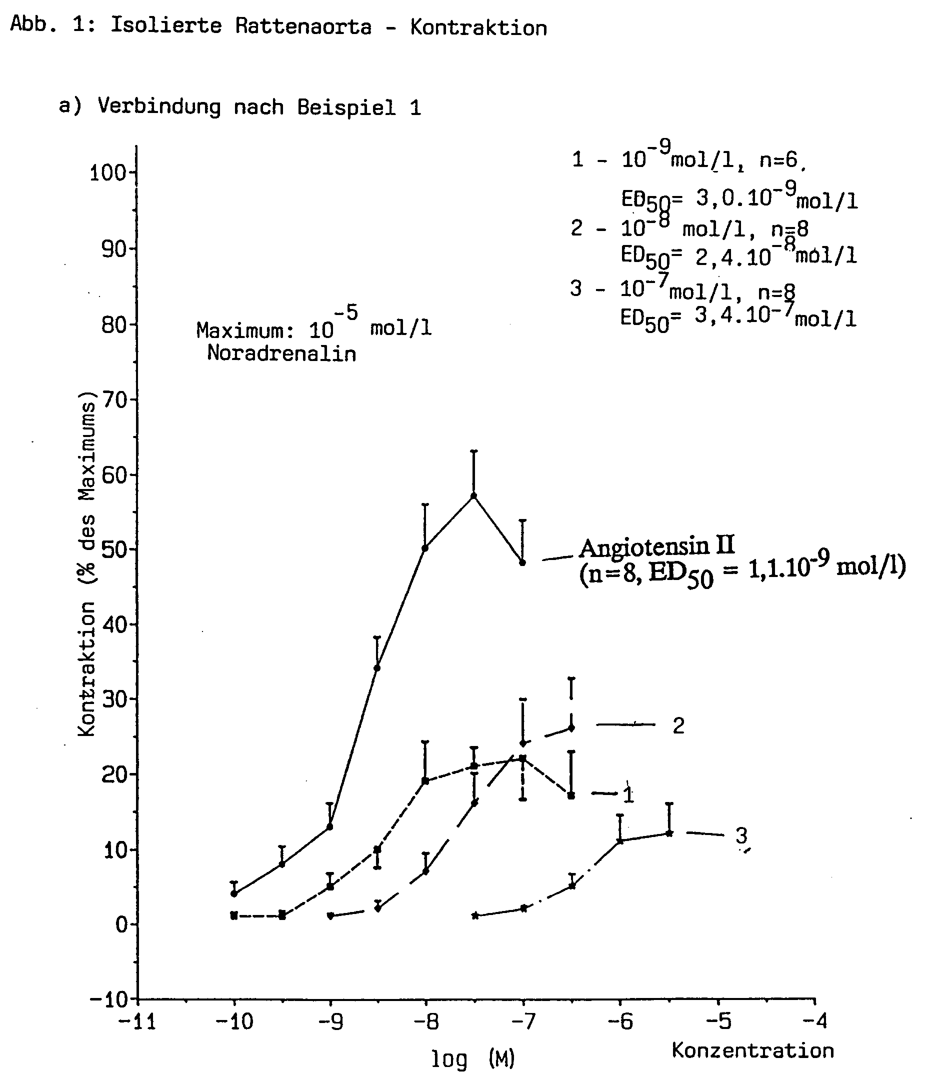 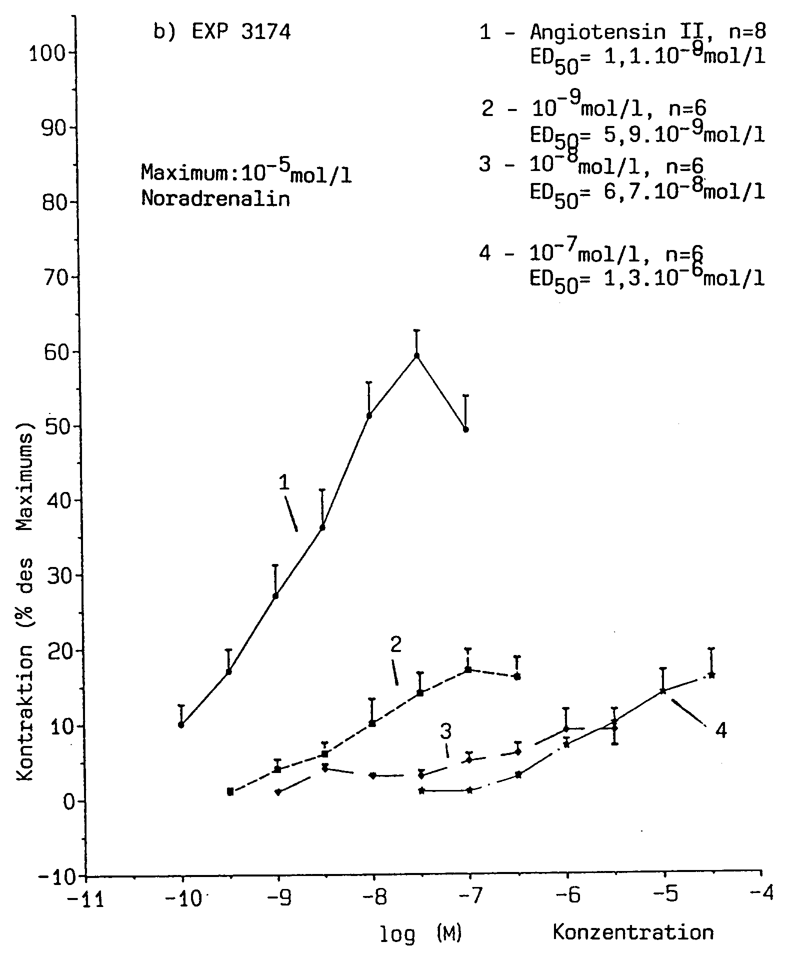
Beide Substanzen reduzierten die maximale Kontraktion durch Angiotensin II dosisbezogen (10⁻⁹ - 10⁻⁷ mol/l), wobei EXP 3174 vergleichsweise stärkter wirkte:

| Isolierte Rattenaorta: % Hemmung der maximalen Kontraktion durch Angiotensin II (3 x 10⁻⁷ mol/l): | | |
|---|---|---|
| mol/l | Verbindung gemäß Beispiel 1 | EXP 3174 |
| 10⁻⁹ | 63 | 76 |
| 10⁻⁸ | 72 | 95 |
| 10⁻⁷ | 98 | 100 |

Auch an despinalisierten Ratten hemmte die Verbindung gemäß Beispiel 1 und EXP 3174 die blutdrucksteigernde Wirkung von Angiotensin II nicht kompetitiv. Im Gegensatz dazu führte DuP 753 zu einer kompetitiven Hemmung, das heißt zu einer parallelen Rechtsverschiebung der Dosis-Wirkungskurve von Angiotensin II ohne Reduktion des Wirkmaximums (Abb. 2, 3). 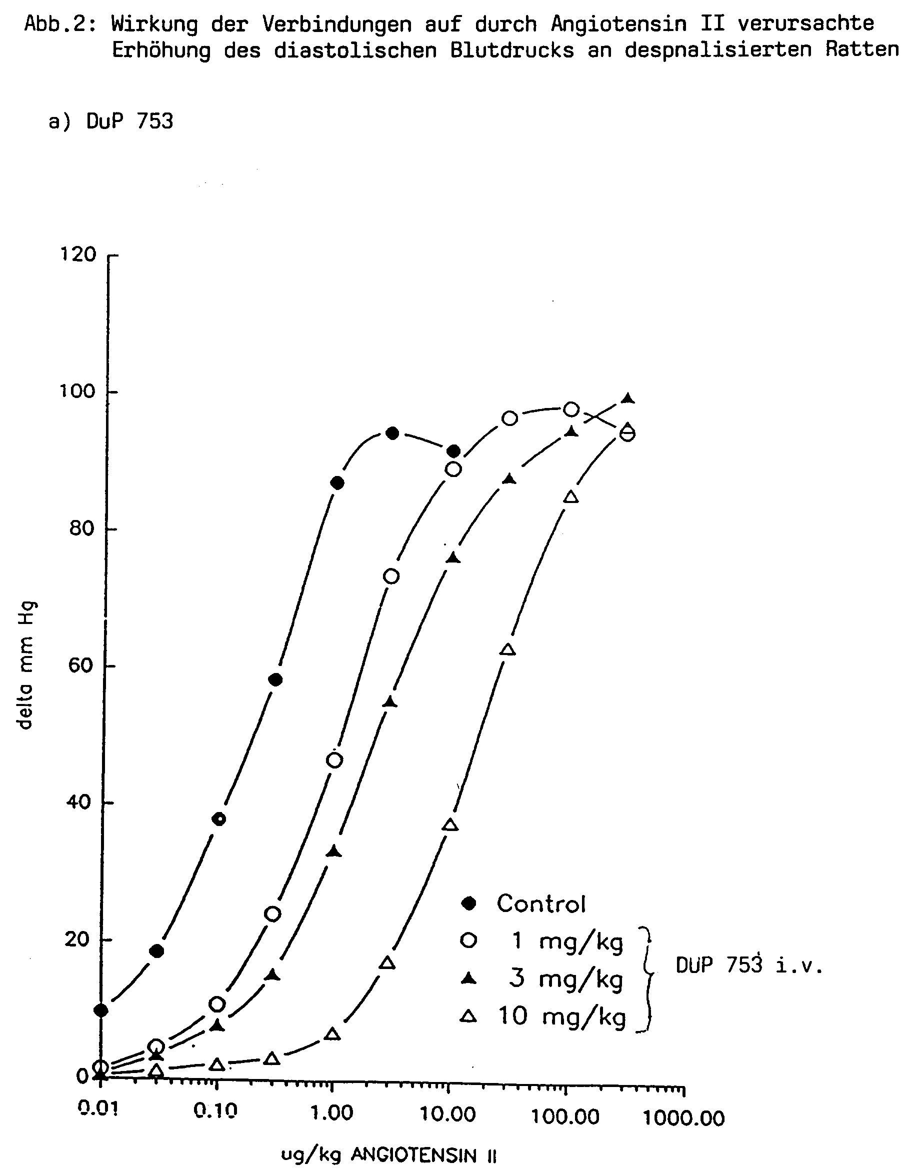 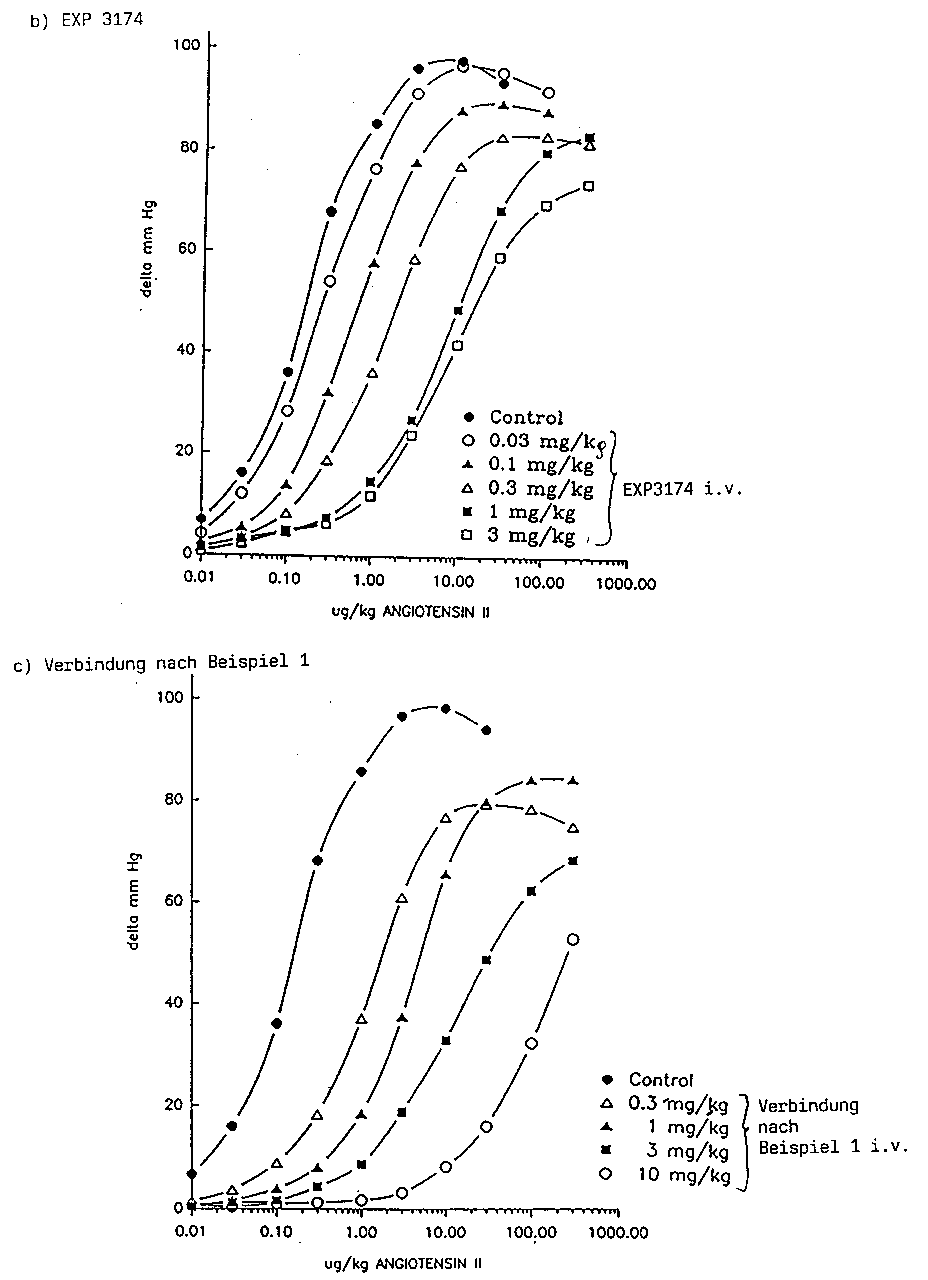 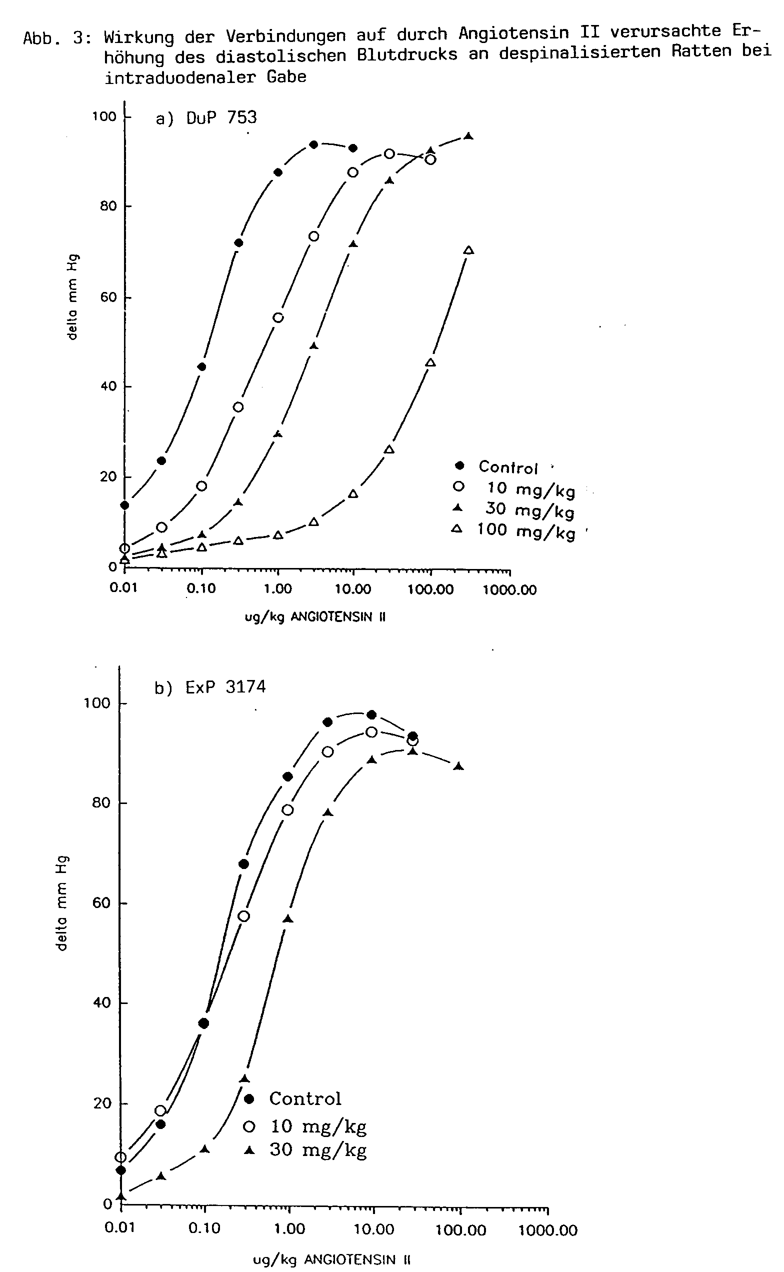 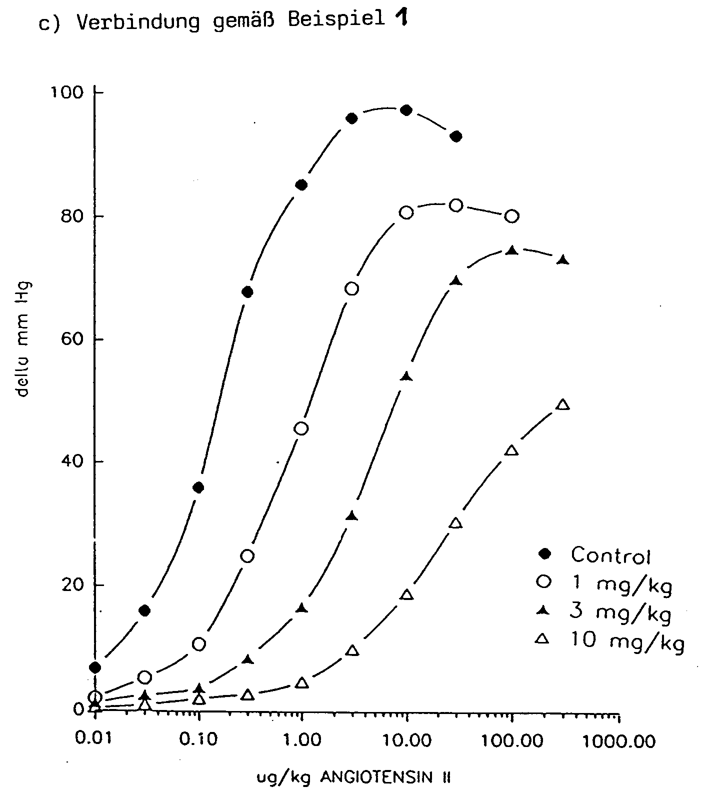
Nach intravenöser Gabe war die Verbindung gemäß Beispiel 1 (0,3 - 10 mg/kg i.v.) leicht potenter als DuP 753 (1 - 10 mg/kg i.v.) und ca. gleich potent wie EXP 3174. Nach intraduodenaler Gabe erwies sich die Verbindung gemäß Beispiel 1 bei weitem als potenteste Substanz. Es ergab sich eine intraduodenale Potenzreihung der Verbindung gemäß Beispiel 1 > DuP 753〉 EXP 3174 (Abb. 4). 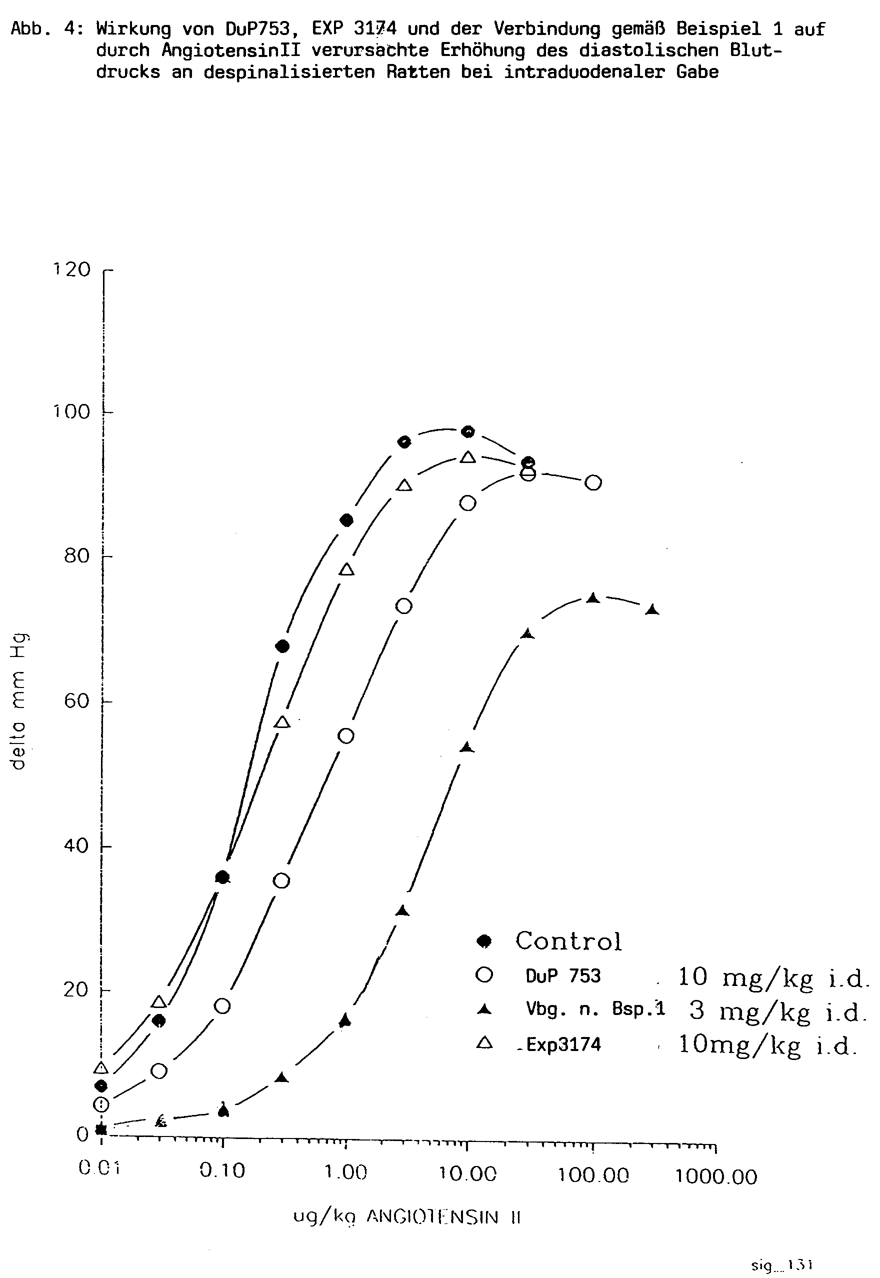
Die Wirkungsdauer der Substanz wurde an narkotisierten, normotensiven Ratten ermittelt. Angiotensin II (1 µg/kg) wurde vor Substanzangabe und in 15 minütigen Intervallen nach intraduodenaler Substanzangabe intravenös appliziert. Wieder ergab sich eine Potenzreihung der Verbindung gemäß Beispiel 1〉 DuP 753. 3 mg/kg der Verbindung gemäß Beispiel 1 waren ca. 2fach effektiver als 10 mg/kg DuP 753 (Abb. 5). Die Verbindung gemäß Beispiel 1 zeigte einen besonders raschen, dosisbezogenen Wirkungseintritt. Die maximale Angiotensin antagonistische Wirkung der Verbindung gemäß Beispiel 1 war bereits nach 15 Minuten nach intradiodenaler Gabe (3 mg/kg) erreicht, im Gegensatz zu 30 - 60 Minuten nach Gabe von DuP 753 (Abb. 6). Die Wirkung der Substanzen blieb über die gesamte Versuchsdauer von 5 Stunden konstant.

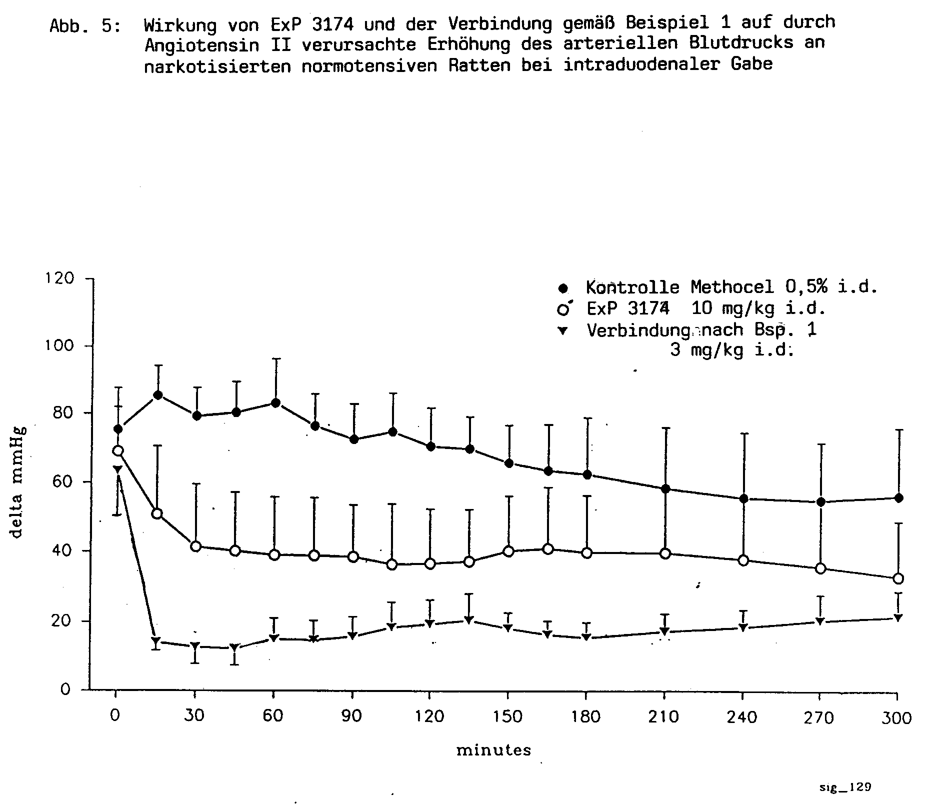

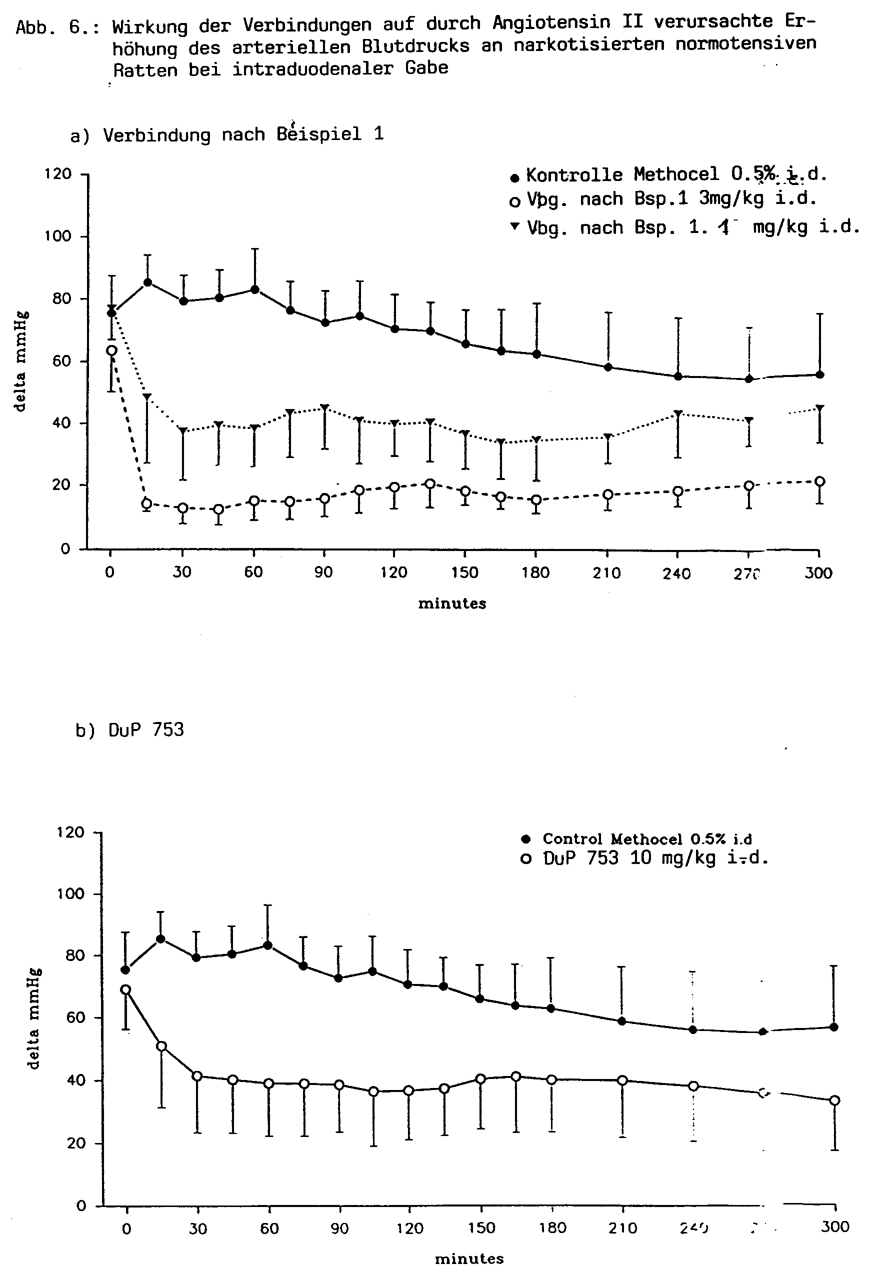

## Patentansprüche

1. Neue Imidazol-5-carbonsäureacylale der allgemeinen Formel 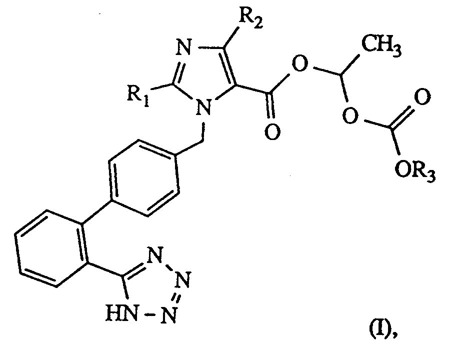 worin R₁ eine gegebenenfalls ungesättigte, geradkettige Alkylgruppe mit 1 - 6 Kohlenstoffatomen, R₂ Wasserstoff, Chlor, Brom, Jod oder CF₃ und R₃ C₁-C₁₀-Alkyl, C₃-C₇-Cycloalkyl oder Benzyl bedeuten sowie ihre pharmazeutisch verträgliche Salze.

2. Verbindungen der in Anspruch 1 definierten allgemeinen Formel (I), worin R₁ Butyl bedeutet.

3. Verbindungen nach Anspruch 2 worin R₂ Chlor bedeutet.

4. 2-Butyl-4-chlor-1-((2'-(1H-tetrazol-5-yl)-biphen-4-yl)-methyl)-1H-imidazol-5-carbonsäure-1-ethoxycarbonylethylester, Natriumsalz.

5. 2-Butyl-4-chlor-1-((2'-(1H-tetrazol-5-yl)-biphen-4-yl)-methyl)-1H-imidazol-5-carbonsäure-1-ethoxycarbonyloxyethylester, Natriumsalz.

6. Ein Verfahren zur Herstellung einer Verbindung nach Anspruch 1, das dadurch gekennzeichnet ist, daß man im Schritt a) eine Verbindung der Formel 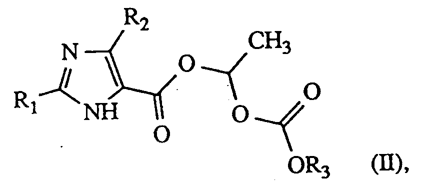 worin R₁, R₂ und R₃ die obige Bedeutung haben mit einer Verbindung der Formel 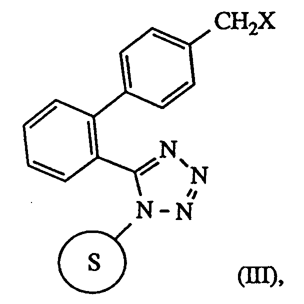 worin X Chlor, Brom oder Jod und 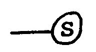 die Triphenylmethylschutzgruppe bedeutet, umsetzt, die so erhaltene Verbindung der Formel 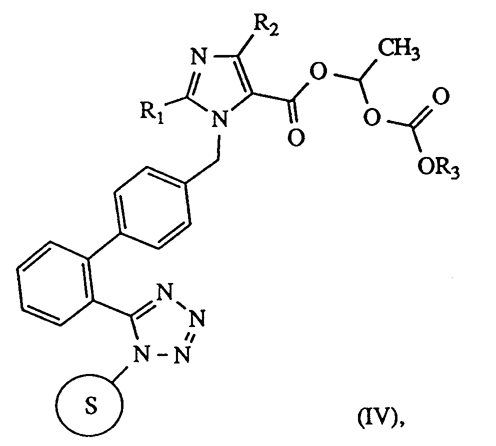 worin R₁, R₂, R₃ und 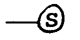 die obige Bedeutung besitzen, im Schritt b) mit einem niederen aliphatischen Alkohol erhitzt und die so erhaltene Verbindung der Formel (I), die wegen ihres amorphen Charakters üblicherweise nicht kristallisiert, gegebenenfalls mit anorganischen oder organischen Basen in ein pharmazeutisch verträgliches Salz überführt.

7. Eine pharmazeutische Zusammensetzung zur oralen Verabreichung, dadurch gekennzeichnet, daß sie eine Verbindung der Formel (I), wie in Anspruch 1 definiert, oder ein pharmazeutisch annehmbares Salz hievon, zusammen mit einem pharmazeutisch annehmbaren Träger oder Verdünnungsmittel enthält.

8. Eine pharmazeutische Zusammensetzung nach Anspruch 7 in Kombination mit anderen therapeutisch wertvollen Wirkstoffen.

9. Verbindungen nach Anspruch 1 und deren Salze zur Verwendung als Wirkstoffe für Arzneimittel zur Behandlung von Krankheiten, die durch Hemmung von Angiotensin II gelindert oder geheilt werden können.

10. Verbindungen nach Anspruch 1 und deren Salze als blutdrucksenkende Heilmittel.

## Patentansprüche (Patentansprüche für folgende(n) Vertragsstaat(en): ES, GR)

1. Verfahren zur Herstellung neuer Imidazol-5-carbonsäureacylale der allgemeinen Formel 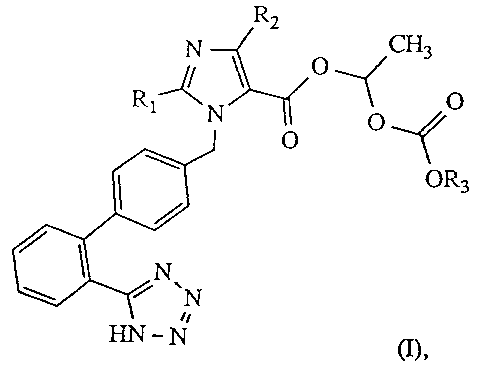 worin R₁ eine gegebenenfalls ungesättigte, geradkettige Alkylgruppe mit 1 - 6 Kohlenstoffatomen, R₂ Wasserstoff, Chlor, Brom, Jod oder CF₃ und R₃ C₁-C₁₀-Alkyl, C₃-C₇-Cycloalkyl oder Benzyl bedeuten sowie ihrer pharmazeutisch verträglichen Salze, das dadurch gekennzeichnet ist, daß man im Schritt a) eine Verbindung der Formel 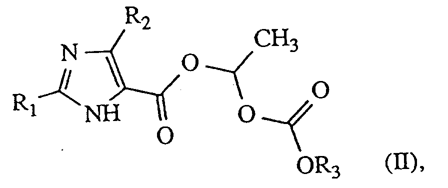 worin R₁, R₂ und R₃ die obige Bedeutung haben mit einer Verbindung der Formel 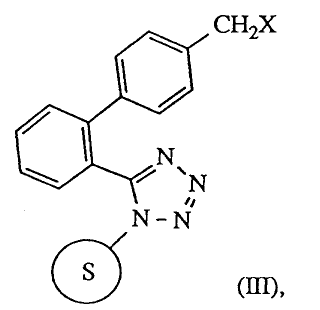 worin X Chlor, Brom oder Jod und 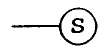 die Triphenylmethylschutzgruppe bedeutet, umsetzt, die so erhaltene Verbindung der Formel 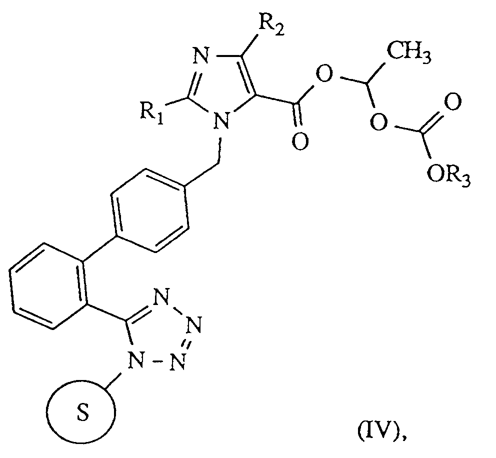 worin R₁, R₂, R₃ und 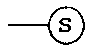 die obige Bedeutung besitzen, im Schritt b) mit einem niederen aliphatischen Alkohol erhitzt und die so erhaltene Verbindung der Formel (I), die wegen ihres amorphen Charakters üblicherweise nicht kristallisiert , gegebenenfalls mit anorganischen oder organischen Basen in ein pharmazeutisch verträgliches Salz überführt.

2. Verfahren nach Anspruch 1, dadurch gekennzeichnet, daß die Schutzgruppe durch Erhitzen mit niederen aliphatischen Alkoholen abgespalten wird.

3. Verfahren nach Anspruch 1, dadurch gekennzeichnet, daß als Schutzgruppe eine Triphenylmethylgruppe verwendet wird.

4. Verfahren zur Herstellung von Arzneimitteln, dadurch gekennzeichnet, daß mindestens eine Verbindung der Formel I hergestellt nach Anspruch 1 gegebenenfalls mit anderen therapeutisch wertvollen Wirkstoffen sowie üblichen galenischen Hilfs- und/oder Trägerstoffen vermischt wird.

5. Verwendung von Verbindungen der Formel I hergestellt nach Anspruch 1 als blutdrucksenkende Arzneimittel.
